# EUROPEAN PATENT APPLICATION

(11) **EP 0 661 050 A1**
(43) Date of publication of application: **05.07.1995**
(21) Application number: 92913545.7
(22) Date of filing: 03.07.1992
(51) Int. Cl.: A61K 31/215

(54) **ANALGESIC**

(30) Priority: 04.07.1991 JP 164525/91
(71) Applicant: TAISHO PHARMACEUTICAL CO. LTD, Tokyo 171 (JP)
(72) Inventor: KANAZAWA, Masakazu, Kodaira-shi, Tokyo 187 (JP)
(74) Representative: Weisert, Annekäte, Dipl.-Ing. Dr.-Ing.
(86) International application number: JP9200843
(87) International publication number: WO9300895

(57) **Abstract**

An analgesic composition which can reduce the side effects caused by ibuprofen, such as the disturbance of a gastric mucous membrane, by adding dicyclomine hydrochloride to ibuprofen to potentiate the analgesic activity.

## Description

### TECHNICAL FIELD

The present invention relates to an analgesic preparation, and more particularly to an analgesic preparation which is characterized by containing ibuprofen and dicyclomine hydrochloride in combination as effective ingredients.

### BACKGROUND ART

Ibuprofen is a known non-steroidal anti-inflammatory, analgesic and antipyretic agent, and is widely used for anti-inflammation and analgesia of rheumatism, arthritis, neuralgia, pharyngitis, uterine adnexitis, dysmenorrhea, cold syndrome, operation and injury.

On the other hand, dicyclomine hydrochloride is known to not only have an atropine-like action which competitively antagonizes acetylcholine and a papaverine-like action which non-competitively antagonizes acetylcholine at the parasympathetic nerve ending, but also have an antispastic action.

However, it is not known that the combination of ibuprofen and dicyclomine hydrochloride enhances the analgesic action.

While gastrointestinal disorders caused by non-steroidal anti-inflammatory agents have been well known in the past, the disorders caused by ibuprofen were recognized to occur with higher incidence than other disorders. These gastrointestinal disorders include nausea, vomiting, angular stomatitis, anorexia, heartburn, upper abdominal pain and diarrhea, and occasionally gastrointestinal bleeding.

Furthermore, menstrual pain (functional dysmenorrhea) is considered to be due to the production of excess prostaglandin or the uterine contraction caused by prostaglandin. Therefore, there is a need for development of drugs which enhance the analgesic action of ibuprofen against the factors which cause these menstrual pain and reduce the accompanying symptoms (e.g. irritation, depressive mood, diarrhea, drowsiness and vomiting).

### DISCLOSURE OF THE INVENTION

In view of the above problems, the present inventor has conducted extensive research in order to reduce the incidence of gastrointestinal disorders caused by analgesic preparations containing ibuprofen, and consequently, has discovered that the analgesic effect is enhanced by combining ibuprofen and dicyclomine hydrochloride and that the accompanying symptoms (e.g. irritation, depressive mood, diarrhea, drowsiness and vomiting) are reduced, and thus has accomplished the present invention.

The present invention is an analgesic preparation which is characterized by containing ibuprofen and dicyclomine hydrochloride in combination as effective ingredients.

According to the analgesic composition of the present invention, ibuprofen and dicyclomine hydrochloride may be formulated into various single unit dosage forms, which can be selected according to the purpose of the therapy. Ibuprofen and dicyclomine hydrochloride may be also formulated individually into separate dosage forms. These dosage forms include forms for oral administration such as powders, syrups, tablets and granules, and forms for parenteral administration such as injectional solutions and suppositories.

These dosage forms can be prepared by using known carriers in the art according to the conventional practices in the art.

Examples of other ingredients which can be contained in the present invention are allylisopropylacetylurea, bromvalerylurea, caffeine and sodium benzoate, caffeine, vitamins, hesperidin, and crude drugs (e.g. lumbricus, glycyrrhiza licorice root, cinnamon bark, peony root, mountan bark, Japanese valerian, zanthoxylum fruit, ginger and citrus unshiu peel). Examples of the carriers are fillers (e.g. lactose, glucose, mannitol and crystalline cellulose), disintegrators (e.g. potato starch, corn starch and carboxymethylcellulose), lubricants (e.g. talc and magnesium stearate), binders (e.g. gelatin, gum arabic, polyvinyl alcohol and hydroxypropylcellulose), all of which can be normally used to prepare dosage forms for oral administration. If necessary, corrigents, cosmetics and antiseptics may be added.

While the daily oral dose of ibuprofen alone which is one of the effective ingredients of the present invention is usually about 600 mg - 1200 mg in three divided doses per adult, the daily dose of ibuprofen contained in the analgesic preparation of the present invention is preferably from 200 mg to 450 mg. On the other hand, dicyclomine hydrochloride is generally administered orally at a dose of 10 - 20 mg per adult, 3 or 4 times a day.

### BEST MODE FOR CARRYING OUT THE INVENTION

The present invention is illustrated in more detail by the following examples and experiments.

### Example 1

| (Formulation 1) | |
|---|---|
| Ibuprofen | 150 mg |
| Dicyclomine hydrochloride | 10 mg |
| Bromvalerylurea | 200 mg |
| Crystalline cellulose | 300 mg |
| Corn starch | 300 mg |
| Hydroxypropylcellulose | 40 mg |
| | 1000 mg/pack |

Granules were prepared at the above-mentioned combination ratio.

### Example 2

| (Formulation 2) | |
|---|---|
| Ibuprofen | 150 mg |
| Dicyclomine hydrochloride | 10 mg |
| Anhydrous caffeine | 50 mg |
| Crystalline cellulose | 350 mg |
| Lactose | 400 mg |
| Hydroxypropylcellulose | 20 mg |
| | 1000 mg/capsule |

Granules were prepared at the above-mentioned combination ratio.

### Example 3

| (Formulation 3) | |
|---|---|
| Ibuprofen | 150 mg |
| Dicyclomine hydrochloride | 10 mg |
| Vitamin B₁ | 4 mg |
| Crystalline cellulose | 400 mg |
| Potato starch | 376 mg |
| Hydroxypropylcellulose | 30 mg |
| | 1000 mg/pack |

Granules were prepared at the above-mentioned combination ratio.

### Example 4

| (Formulation 4) | |
|---|---|
| Ibuprofen | 150 mg |
| Dicyclomine hydrochloride | 10 mg |
| Glycyrrhiza licorice root | 500 mg |
| Crystalline cellulose | 220 mg |
| Corn starch | 100 mg |
| Hydroxypropylcellulose | 20 mg |
| | 1000 mg/pack |

Granules were prepared at the above-mentioned combination ratio.

### Example 5

| (Formulation 5) | |
|---|---|
| Ibuprofen | 150 mg |
| Dicyclomine hydrochloride | 10 mg |
| Anhydrous caffeine | 100 mg |
| Bromvalerylurea | 150 mg |
| Crystalline cellulose | 350 mg |
| Mannitol | 300 mg |
| Hydroxypropylcellulose | 40 mg |
| | 1000 mg/pack |

Granules were prepared at the above-mentioned combination ratio.

### Example 6

| (Formulation 6) | |
|---|---|
| Ibuprofen | 150 mg |
| Dicyclomine hydrochloride | 10 mg |
| Anhydrous caffeine | 100 mg |
| Bromvalerylurea | 150 mg |
| Vitamin B₁ | 5 mg |
| Crystalline cellulose | 250 mg |
| Lactose | 300 mg |
| Hydroxypropylcellulose | 35 mg |
| | 1000 mg/pack |

Granules were prepared at the above-mentioned combination ratio.

### Experiment 1

### (Test drugs)

Test drug 1: Ibuprofen (concentrations corresponding to 5.0, 10.0, 20.0 and 40.0 mg/kg)
Test drug 2: Dicyclomine hydrochloride (concentrations corresponding to 13.0 and 27.0 mg/kg)
Test drug 3: Composition of ibuprofen and dicyclomine hydrochloride at a ratio of 15:1 (concentrations corresponding to 2.7, 5.4, 10.7 and 21.3 mg/kg)
Test drug 4: The desired amount of the granules of Example 1 was weighed, ground in a mortar, and suspended in a 0.5% aqueous tragacanth gum solution in the concentrations corresponding to 2.5, 5.0, 10.0 and 20.0 mg/kg.

Test drug 5: The desired amount of the granules of the following Formulation 7 was weighed, ground in a mortar, and suspended in a 0.5% aqueous tragacanth gum solution in the concentrations corresponding to 50.0, 100.0, 200.0 and 400.0 mg/kg.

| (Formulation 7) | |
|---|---|
| Ibuprofen | 150 mg |
| Bromvalerylurea | 200 mg |
| Crystalline cellulose | 300 mg |
| Corn starch | 310 mg |
| Hydroxypropylcellulose | 40 mg |
| | 1000 mg/pack |

The dose was 10 ml/kg.

### (Test animal)

Male Wistar strain rats each weighing 245 - 380 g were subjected to a vaginal fur test to select those rats which were in the resting stage.

### (Test method)

Animals were anesthetized with urethane (1.0 g/kg, i.p.) and fixed dorsally. An open-end catheter was inserted into one side of the uterine bicornis through the uterine cervix, and the resting internal pressure was adjusted to about 10 cm H₂O by adding saline solution. The change in internal pressure was leaded to an amplifier of distortion pressure (Nippon Koden, AP-601G) via a pressure transducer (Nippon Koden, TP-300T) and recorded on recorders (National, VP-6621A and YOKOGAWA, 3306). When the uterine movement became stable, the test drug was administered duodenally via a previously inserted catheter, and the change in internal pressure was recorded for 30 minutes after administration (120 minutes in cases where a response was recognized). ID₅₀ value was calculated from each frequency of uterine movement at both 30 minutes before administration, and 30 minutes after administration according to the Litchfield-Wilcoxon method.

### (Result)

Results are shown in Table 1.

**Table 1**

| Test drug No. | ID₅₀ value (mg/kg) |
|---|---|
| 1 | 22.3 |
| 2 | >27 |
| 3 | 10.7 |
| 4 | 8.0 |
| 5 | 256.5 |

### Experiment 2

### (Test drug)

Test drug 6: The desired amount of the granules of Example 1 was weighed, ground in a mortar, and suspended in a 0.5% aqueous tragacanth gum solution in the desired concentrations.

Test drug 7: The desired amount of the granules of the following Formulation 8 was weighed, ground in a mortar, and suspended in a 0.5% aqueous tragacanth gum solution in the desired concentrations.

| (Formulation 8) | |
|---|---|
| Ibuprofen | 300 mg |
| Bromvalerylurea | 200 mg |
| Crystalline cellulose | 150 mg |
| Corn starch | 310 mg |
| Hydroxypropylcellulose | 40 mg |
| | 1000 mg/pack |

The dose was 10 ml/kg.

The preparation was carried out by following a similar method to that of Experiment 1.

### (Test animals)

Groups of ten male Wistar strain rats each weighing 183 - 202 g which were made to fast for 24 hours but which were given free access to water, were used for each test drug.

### (Test schedule)

The test drug was administered orally, and the gastric mucosal disorders were observed 4 hours after administration.

The test was primarily carried out according to the method of A. Robert et al as described in Gastroenterology, vol. 77, pp. 433 - 443 (1979).

In order to facilitate the observation of the gastric mucosal disorders, a 4% brilliant blue 6b (Tokyo Kasei) saline solution was administered via the caudal vein 3 hours after administration of the test drug. The removed stomach was cut open along the greater curvature, and the incidence and the length of ulcers were determined.

### (Results)

Results are shown in Table 2

**Table 2**

| Test drug No. | Number of incidence/number of animal | Ulcer coefficient (nm) Means ± Standard deviation |
|---|---|---|
| 6 | 4/10 | 5.3 ± 3.4 |
| 7 | 9/10 | 6.4 ± 2.5 |

Ulcers did not break out in the control group. When 300 mg/kg of the test drug was administered, the intensity of ulcers by the ulcer coefficient of Test drug 6 was significantly weaker than that of Test drug 7.

### INDUSTRIAL APPLICABILITY

The present invention provides an analgesic preparation which makes it possible to reduce the prior content of analgesic drug, and lowers the incidence of side effects.

## Claims

1. An analgesic composition which is characterized by containing ibuprofen and dicyclomine hydrochloride in combination as effective ingredients.

2. Use of a composition comprising ibuprofen and dicyclomine hydrochloride as effective ingredients for the manufacture of an analgesic preparation.

3. A kit for an analgesic preparation comprising ibuprofen and dicyclomine hydrochloride which are formulated into separate forms.
